# EUROPEAN PATENT APPLICATION

(11) **EP 4 512 333 A1**
(43) Date of publication of application: **26.02.2025**
(21) Application number: 24193299.5
(22) Date of filing: 07.08.2024
(51) Int. Cl.: A61B 5/15, A61B 5/00, A61B 5/145

(54) **WEARABLE DEVICE FOR ON-DEMAND INTERSTITIAL FLUID BIOMARKER SENSING WITH A MICRONEEDLE-INTEGRATED SENSOR**

(30) Priority: 25.08.2023 US 202318456146
(71) Applicant: General Electric Company, Schenectady, NY 12345 (US)
(72) Inventor: BURNS, Andrew Arthur Paul, Niskayuna, 12309 (US); ALIZADEH, Azar, Niskayuna, 12309 (US); LENIGK, Ralf, Niskayuna, 12309 (US); ST. PIERRE, Richard JeanLuc, Niskayuna, 12309 (US)
(74) Representative: Kilburn & Strode LLP

(57) **Abstract**

A microneedle actuator (108) for monitoring ISF of a user includes a rigid support (112) defining a slot (130), a geared cam (118) positioned within the rigid support (112) and including an axle (147) extending through the slot (130), and a spring (114) positioned within the rigid support (112) and configured to rotate the geared cam (118) about the axle (147). The microneedle actuator (108) further includes an ISF sensing module (120) and an actuation device (122). The ISF sensing module (120) is attached to the geared cam (118) and including a plurality of microneedles (214), each microneedle of the plurality of microneedles (214) including at least one sensor (244) for detecting the ISF of the user. The actuation device (122) is attached to the rigid support (112) and the geared cam (118). Actuation of the actuation device (122) releases the spring (114), rotating the geared cam (118) to insert the plurality of microneedles (214) into the user's skin, and wherein the at least one sensor (244) detects constituents of the ISF of the user.

## Description

### STATEMENT REGARDING FEDERALLY SPONSORED RESEARCH AND DEVELOPMENT

This invention was made with Government support under a Development Agreement supported by an award by the Air Force Research Laboratory. The Government has certain rights in this invention.

### BACKGROUND

The subject matter described herein relates generally to wearable sensing systems and, more particularly, to wearable systems for sensing interstitial fluid of a user.

Continuous monitoring of biochemical data, or biomarkers, may be important for certain medical conditions. For example, continuous monitoring of biomarkers may enable a user being monitored or a medical professional to medically intervene when the biomarkers indicate that medical intervention could be advantageous and/or necessary to properly manage a medical condition. Poor management of these medical conditions may result in short-term or long-term injury to the user. Similarly, biomarkers may be monitored to evaluate the performance of a user during athletic, military, or cognitive activities. The ability to detect and quantify biomarkers in situ and in real-time enables the user or observers to implement changes in the short-term or long-term to augment performance or prevent degradation of performance (e.g., maintaining hydration, ensuring proper rest, increasing or decreasing training/task load, administering of pharmaceuticals or supplements to enhance performance, etc.).

At least some known biochemical monitoring devices monitor the blood of the user. These biochemical monitoring devices typically include at least one needle or microneedle that penetrates the skin of the user and channel the blood to sensing devices that are located on the surface of the skin. The sensing devices detect and report the presence and/or concentration of biomarkers in the blood to the user or the medical professional. However, because the sensing devices are located on the surface of the skin, the blood is typically continually replenished and must be disposed of after the sensing devices have analyzed the blood. Typically, a wick absorbs the blood, and the wick is discarded after the wick cannot absorb any more blood. As such, the wick is typically frequently replaced in order to continually monitor and analyze the blood. Additionally, these biochemical monitoring devices may be difficult for a user to use due to blood clots (e.g., the extracted blood may clot within the biochemical monitoring device). Moreover, these biochemical monitoring devices may be painful for the user to use because the needle extends through nerves within the skin.

At least some known biochemical monitoring devices monitor the ISF of the user. For certain medical conditions, the concentration of the biomarkers in interstitial fluid (ISF) is correlated to the concentration of the biomarkers in the blood of the user. Because ISF is not excreted, ISF is not contaminated by the environment and does not clot, making it an excellent source for continuous and on-demand biomarker monitoring. These biochemical monitoring devices typically include at least one needle or microneedle that penetrates the skin of the user and monitors the ISF of the user subcutaneously and continuously. These biochemical monitoring devices typically include at least one needle or microneedle that penetrates the skin of the user and at least one sensing device that is located on the microneedle and penetrates the skin with the microneedle. The microneedle and the sensing device remain in the skin of the user for an extended period of time to continuously monitor the ISF of the user. However, microneedles and sensor devices inserted in the skin may cause inflammatory and/or foreign body responses which may introduce artifacts in inflammatory cytokine measurements. Additionally, for protein biomarker sensing, extremely high affinity binding biorecognition elements (BREs) are used, which are used for single point measurements because they may not respond effectively to decreases in concentration (de-binding). The inflammation and foreign body responses caused by the microneedles and the sensing devices may distort the results reported by the BREs.

### BRIEF DESCRIPTION

In one aspect a microneedle actuator for monitoring ISF of a user is provided. The microneedle actuator includes a rigid support defining a slot, a geared cam positioned within the rigid support and including an axle extending through the slot, and a spring positioned within the rigid support and configured to rotate the geared cam about the axle. The microneedle actuator further includes an ISF sensing module and an actuation device. The ISF sensing module is attached to the geared cam and including a plurality of microneedles, each microneedle of the plurality of microneedles including at least one sensor for detecting the ISF of the user. The actuation device is attached to the rigid support and the geared cam. Actuation of the actuation device releases the spring, rotating the geared cam to insert the plurality of microneedles into the user's skin, and wherein the at least one sensor detects constituents of the ISF of the user.

In another aspect a microneedle actuator for monitoring ISF of a user. The microneedle actuator includes a rigid support defining a slot, a geared cam positioned within the rigid support and including an axle extending through the slot, a spring positioned within the rigid support and configured to rotate the geared cam about the axle, and an ISF sensing module attached to the geared cam. The ISF sensing module includes a first support layer, a sensor layer laminated to the first support layer to form a sensor component, and a plurality of microneedles extending from the sensor component. The sensor layer includes a substrate and at least one sensor positioned on the substrate. Each microneedle of the plurality of microneedles including a microneedle base extending from the sensor component, a body, and a tip extending from the body. The body extending from the microneedle base and defining a slot extending through the first support layer of the body. The slot defining a first opening and a second opening, the at least one sensor positioned over the second opening, where the first opening, the slot, and the at least one sensor define a channel exposed to the environment, where the channel is configured to channel ISF to the at least one sensor. The ISF sensing module including an actuation device attached to the rigid support and the geared cam, wherein actuation of the actuation device releases the spring, rotating the geared cam to insert the plurality of microneedles into the user's skin, and where the at least one sensor detects the ISF of the user.

In yet another aspect, A method of detecting a biomarker within the ISF of a user is provided. The method includes adhering a microneedle actuator to the user's skin, the microneedle actuator including a rigid support, a geared cam positioned within the rigid support, a spring positioned with the rigid support and configured to rotate the geared cam, an ISF sensing module attached to the geared cam and including a plurality of microneedles, and an actuation device attached to the rigid support and the geared cam. Each microneedle of the plurality of microneedles including at least one sensor for detecting the ISF of the user. The method further includes actuating the actuation device such that the actuation device releases the spring, rotating the geared cam to insert the plurality of microneedles into the user's skin. The method also includes channeling the ISF to the at least one sensor and detecting at least one biomarker with the at least one sensor.

### DRAWINGS

These and other features, aspects, and advantages of the present disclosure will become better understood when the following detailed description is read with reference to the accompanying drawings in which like characters represent like parts throughout the drawings, wherein:
FIG. 1 is a top perspective view of an exemplary wearable ISF sensing device.
FIG. 2 is a cut-away perspective view of an exemplary microneedle actuator shown in FIG. 1.
FIG. 3 is a side view of the exemplary microneedle actuator shown in FIG. 2.
FIG. 4 is a side view of the exemplary microneedle actuator shown in FIG. 2 with portions removed for clarity.
FIG. 5 is a side view of the exemplary microneedle actuator shown in FIG. 2 with portions illustrated as transparent for clarity.
FIG. 6 is a side view of the exemplary microneedle actuator shown in FIG. 2 with portions illustrated as transparent for clarity.
FIG. 7 is a side view of an exemplary actuation device for use with the microneedle actuator shown in FIG. 2.
FIG. 8 is a top view of an exemplary actuation device for use with the microneedle actuator shown in FIG. 2.
FIG. 9 is a plan view of an exemplary ISF sensing module.
FIG. 10 is an exploded perspective view of the ISF sensing module shown in FIG. 9.
FIG. 11 is a plan view of a top support layer of the ISF sensing module shown in FIG. 9.
FIG. 12 is a plan view of a bottom support layer of the ISF sensing module shown in FIG. 9.

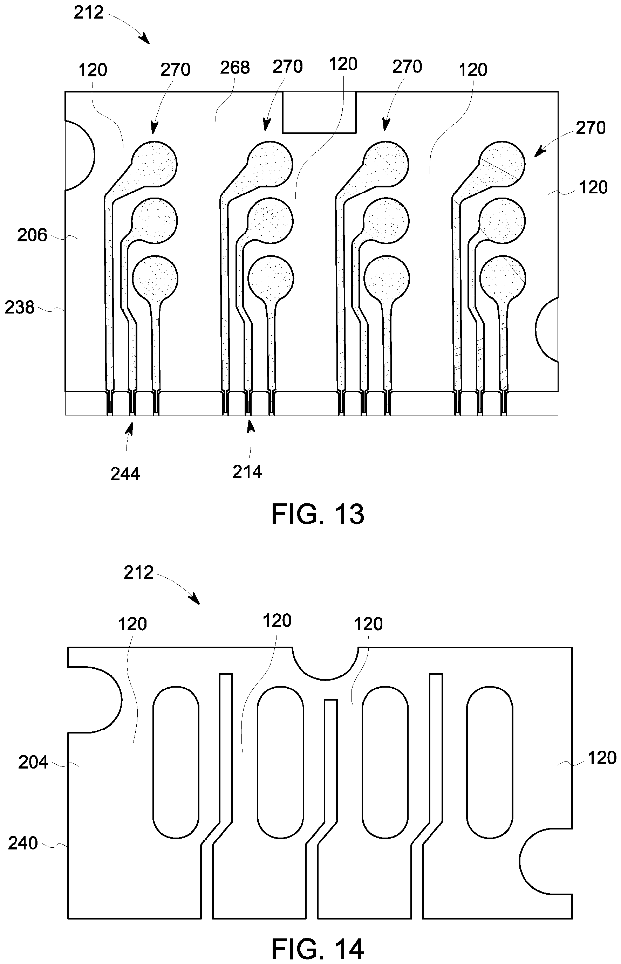

FIG. 13 is a plan view of an sensor layer of the ISF sensing module shown in FIG. 9.
FIG. 14 is a plan view a first adhesive layer of the ISF sensing module shown in FIG. 9.
FIG. 15 is a plan view of a second adhesive layer of the ISF sensing module shown in FIG. 9.
FIG. 16 is an exploded perspective view of a microneedle of the ISF sensing module shown in FIG. 9.
FIG. 17 is a perspective view of the top support layer of the microneedle shown in FIG. 16.
FIG. 18 is a plan view of the top support layer of the microneedle shown in FIG. 16.

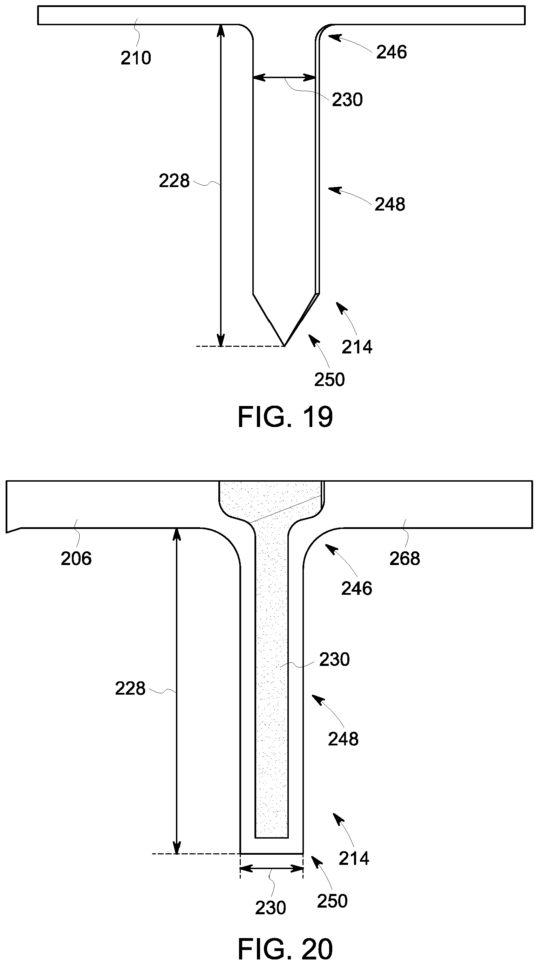

FIG. 19 is a plan view of the bottom support layer of the microneedle shown in FIG. 16.
FIG. 20 is a plan view of the sensor layer of the microneedle shown in FIG. 16.
FIG. 21 is a block diagram of an exemplary electronics architecture.
FIG. 22 is an electrical diagram of a plurality of sensors printed on a substrate of the sensor layer shown in FIG. 13.
FIG. 23 is a diagram of the wearable ISF sensing device shown in FIG. 1 positioned on a user's skin with the microneedles shown in FIG. 16 inserted into the dermis of the user's skin.

Unless otherwise indicated, the drawings provided herein are meant to illustrate features of embodiments of the disclosure. These features are believed to be applicable in a wide variety of systems comprising one or more embodiments of the disclosure. As such, the drawings are not meant to include all conventional features known by those of ordinary skill in the art to be required for the practice of the embodiments disclosed herein.

### DETAILED DESCRIPTION

In the following specification and the claims, reference will be made to a number of terms, which shall be defined to have the following meanings.

The singular forms "a", "an", and "the" include plural references unless the context clearly dictates otherwise.

Approximating language, as used herein throughout the specification and claims, may be applied to modify any quantitative representation that could permissibly vary without resulting in a change in the basic function to which it is related. Accordingly, a value modified by a term or terms, such as "about," "substantially," and "approximately," are not to be limited to the precise value specified. In at least some instances, the approximating language may correspond to the precision of an instrument for measuring the value. Here and throughout the specification and claims, range limitations may be combined and/or interchanged, such ranges are identified and include all the sub-ranges contained therein unless context or language indicates otherwise.

The systems and methods described herein provide a wearable interstitial fluid (ISF) sensing device. The wearable ISF sensing device includes a sensor component and a plurality of microneedle actuators positioned on the sensor component. The sensor component is attached to the skin of a user. The microneedle actuators each include a rigid support, a spring, a ram, a geared cam, an ISF sensing module, and an actuation device. The ISF sensing module is attached to the geared cam. The ram, the spring, and the actuation device are configured to rotate the geared cam to insert the ISF sensing module into the skin of the user. Specifically, the actuation device is configured to maintain the spring in a stored configuration until selectively actuated. Once the actuation device has been actuated, the spring is released from the stored configuration to push, press, or drive the ram against the geared cam, rotating the geared cam. Rotation of the geared cam causes the ISF sensing module to rotate toward the skin of the user, inserting the ISF sensing module into the skin of the user. The ISF sensing module then monitors biochemical markers of the user.

The ISF sensing module includes a plurality of layers laminated together to form a sensor component and at least one microneedle extending from the sensor component. The layers include a first support layer, a first adhesive layer, a sensor layer, a second adhesive layer, and a second support layer. The first and second support layers structurally support the sensor layer, and the first and second adhesive layers laminate the first and second support layers to the sensor layer. Lamination of the layers ensures that the microneedles remain intact as they are inserted into the skin of the user. As such, the microneedles of the ISF sensing modules described herein are structurally stronger than previous microneedles and remain intact as they monitor biomarkers within the user's ISF.

The microneedle includes a microneedle base, a body, and a tip. The microneedle has a substantially flat or planar profile and a slot is defined within the body of the microneedle. The slot has a first opening and a second opening. The sensor layer includes a sensor positioned over the second opening such that the sensor and the slot define a channel that channels ISF to the sensor. The microneedle and the sensor are inserted into the user's skin such that the sensor is positioned within the dermis of the user's skin. The ISF flows into the channel from adjacent tissue, and the sensor detects and measures biomarkers within the ISF. The user, observer, trainer, coach, and/or a medical professional may then monitor a medical condition or a performance/readiness metric based on the data collected by the sensor.

Because the sensors are embedded in the dermis, the ISF that flows to the sensors is continually equilibrated with the surrounding ISF without requiring mechanical mechanisms to replenish the ISF. Additionally, embedding the sensors in the dermis enables the ISF sensing module to detect biomarkers without drawing fluid out of the body of the user, reducing the cleanup and disposal of bodily fluids as the result of biomarker detection and measurement. Moreover, capillary pressure and the compressive force generated during insertion will cause ISF to flow into the channel such that a fluid motive device, such as a vacuum pump or mechanical compression, is not required to replenish the ISF at the sensors. After insertion, passive diffusion from the ISF and equilibration with the ISF inside the channel replenishes the ISF at the sensors. Thus, the ISF sensing module enables detection of biomarkers while reducing cleanup and disposal of bodily fluids and reducing the equipment required to measure biomarkers within the ISF. Additionally, directly measuring ISF in the interstitial space reduces the lag time between changes in biomarker concentration and read-out. Furthermore, the ISF sensing module includes multiple sensors in a single patch that monitor multiple analytes simultaneously and on-demand. For example, the sensors may measure a single analyte multiple times, measure multiple analytes at pre-determined times, measure a secondary analyte after certain conditions for a primary analyte are met, and/or measure a panel of analytes simultaneously.

Because the wearable ISF sensing device includes a plurality of microneedle actuators, the wearable ISF sensing device is capable of monitoring the user's medical condition for a longer period of time compared to bio-sensing devices with a single set of microneedles. Accordingly, a user is not required to replace the wearable ISF sensing device as often as other bio-sensing devices. Additionally, the wearable ISF sensing devices described herein enables consistent monitoring of ISF because the wearable ISF sensing device may automatically activate another microneedle actuator after the sensors of a first microneedle actuator have been consumed. As such, the wearable ISF sensing devices described herein enable consistent monitoring of a user's medical/performance condition. Moreover, because the wearable ISF sensing device includes a plurality of microneedle actuators, the wearable ISF sensing device reduces the number of bio-sensing devices a user consumes to monitor a medical condition.

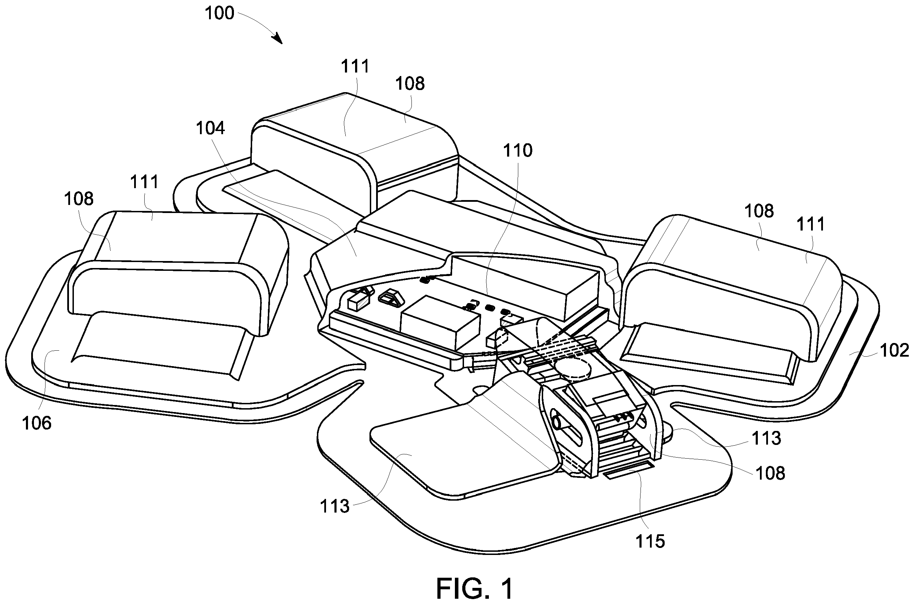

Referring now to the figures, FIG. 1 is a top perspective view of an exemplary embodiment of a wearable ISF sensing device 100. Wearable ISF sensing device 100 includes a sensor component 102 optionally including on-patch electronics 104 and a protective foam 106. Wearable ISF sensing device 100 also includes a plurality of microneedle actuators 108 positioned on sensor component 102 under a plurality of protective covers 111. Wearable ISF sensing device 100 may also include a reusable electronics module 110 positioned on sensor component 102 and electrically coupled to microneedle actuators 108. In the exemplary embodiment, portions of wearable ISF sensing device 100 are disposable (i.e., sensor component 102 and microneedle actuators 108 are generally used once) while portions of wearable ISF sensing device 100 are reusable (i.e., reusable electronics module 110 is generally used more than once). In an alternative embodiment, at least one of sensor component 102 and microneedle actuators 108 are reusable.

Sensor component 102 is attached to the user's skin using an adhesive surface or foam. Alternatively, in some embodiments, sensor component 102 may be integrated into clothing and held in place using a suction device or compression clothing. Sensor component 102 may be affixed to various portions of the user's body, including, but not limited to, the torso, legs, back, neck, arms, etc. For example, sensor component 102 may be placed on the lower back (e.g., above the waistline, below the rib cage, and adjacent to the spine on either side), on the upper back (e.g., above or on the scapula), on the chest (e.g., below the pectoral muscle or centrally over the sternum), on the lower leg (e.g., over the gastrocnemius or on the lateral face over the tibialis anterior muscle), or on the upper leg (e.g., on the inner thigh). Additionally, sensor component 102 includes embedded circuits (not shown) to electrically and communicatively couple on-patch electronics 104, reusable electronics module 110, and/or microneedle actuators 108.

Wearable ISF sensing device 100 may also include clips 113 attached to sensor component 102 for selectively attaching microneedle actuators 108 to sensor component 102. Clips 113 enable microneedle actuators 108 to be replaced after use and sensor component 102 and reusable electronics module 110 to be reused. Protective covers 111 are positioned on sensor component 102 and clips 113 and extend through protective foam 106 to cover, contain, and protect microneedle actuators 108. Protective foam 106 is positioned on sensor component 102 to cover and protect sensor component 102 and reusable electronics module 110. Sensor component 102 defines a plurality of slots 115 positioned proximate each microneedle actuator 108 to enable each microneedle actuator 108 to access the user's skin.

In another alternative embodiment, wearable ISF sensing device 100 may not include sensor component 102. Rather, microneedle actuators 108 may be individually attached to the user's skin and may monitor a medical condition and/or performance metric as described herein. In yet another alternative embodiment, wearable ISF sensing device 100 may not include on-patch electronics 104 and reusable electronics module 110, and may only include sensor component 102 and microneedle actuators 108.

As described herein, microneedle actuators 108 include an actuation mechanism for inserting a microneedle and a sensor into the user's skin such that the sensor is positioned within the dermis of the user's skin. The ISF flows into the sensor and the sensor detects and measures biomarkers within the ISF. The user and/or a medical professional then monitors a medical condition based on the data collected by the sensor. More specifically, each microneedle actuator 108 deploys at least one microneedle on demand such that microneedle actuator 108 collects data for a predetermined amount of time. Microneedle actuator 108 sends the data to on-patch electronics 104 and/or reusable electronics module 110 which then sends the data to the user.

Wearable ISF sensing device 100 may include either on-patch electronics 104 or reusable electronics module 110. Alternatively, wearable ISF sensing device 100 may include both on-patch electronics 104 and reusable electronics module 110, or may not include either on-patch electronics 104 or reusable electronics module 110. Specifically, on-patch electronics 104 and/or reusable electronics module 110 may be configured to receive data from microneedle actuators 108 and send and/or display the data to a user. More specifically, on-patch electronics 104 and/or reusable electronics module 110 may be capable of wireless communication with a remote computing device (e.g., a mobile computing device) using Bluetooth communications or alternative approaches such as direct Wi-Fi. Accordingly, on-patch electronics 104 and/or reusable electronics module 110 may send and/or display collected data to the user.

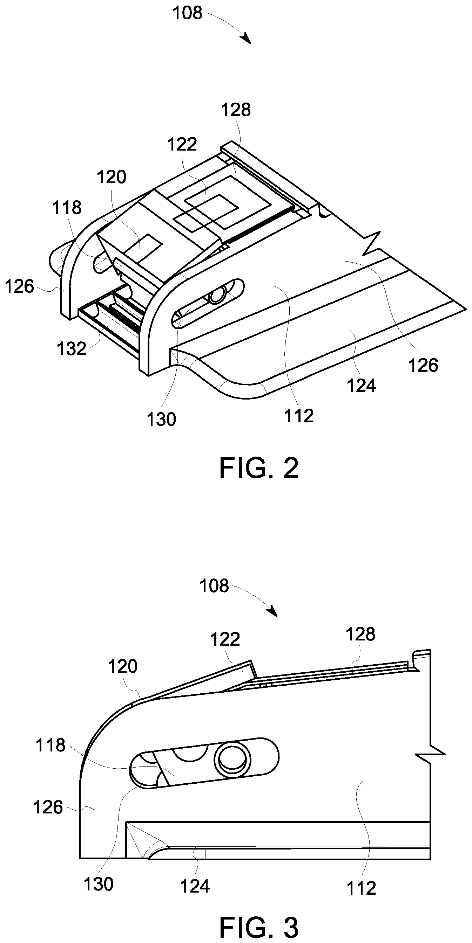

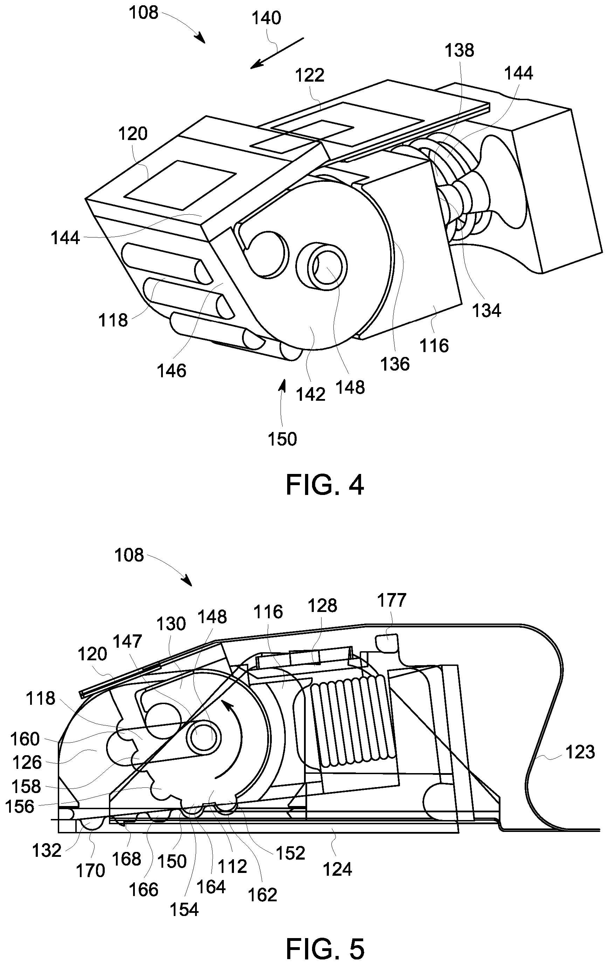

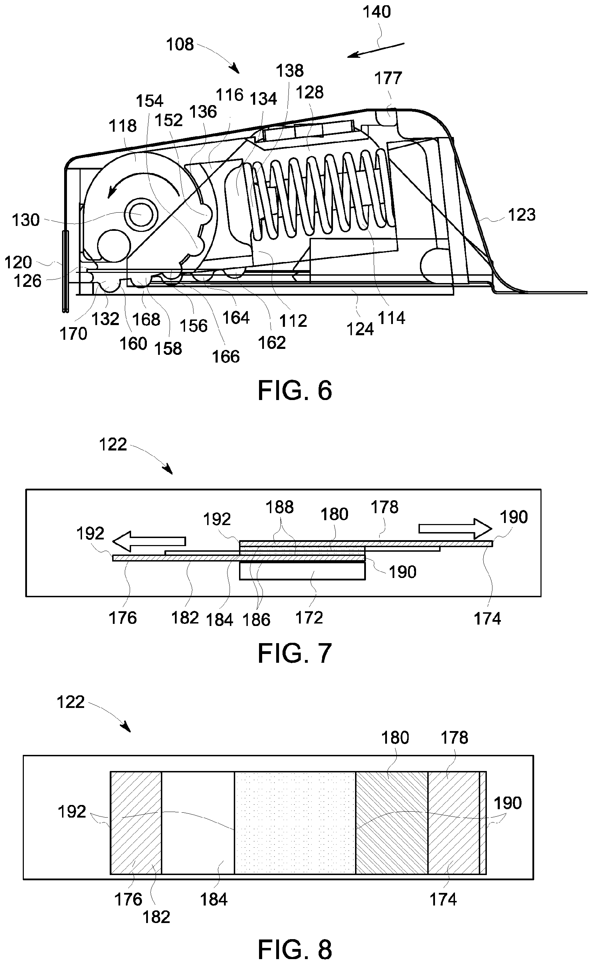

FIG. 2 is a perspective view of microneedle actuator 108. FIG. 3 is a side view of microneedle actuator 108. FIG. 4 is a side view of microneedle actuator 108 with portions of microneedle actuator 108 removed for clarity. FIG. 5 is another side view of microneedle actuator 108 with portions of microneedle actuator 108 illustrated as transparent for clarity. FIG. 6 is another side view of microneedle actuator 108 with portions of microneedle actuator 108 illustrated as transparent for clarity. Microneedle actuator 108 includes a rigid support 112, a spring 114, a ram 116, a geared cam 118, an ISF sensing module 120, an actuation device 122, and a sensor flex connection 123. ISF sensing module 120 is attached to geared cam 118. Ram 116, spring 114, and actuation device 122 are configured to rotate geared cam 118 to insert ISF sensing module 120 into the skin of the user through slots 115. Specifically, actuation device 122 is configured to maintain spring 114 in a stored configuration until selectively actuated. Once actuation device 122 has been actuated, spring 114 is released from the stored configuration to push, press, or drive ram 116 against geared cam 118, rotating geared cam 118. Rotation of geared cam 118 causes ISF sensing module 120 to rotate toward the skin of the user, inserting ISF sensing module 120 into the skin of the user. ISF sensing module 120 then monitors biochemical markers of the user as described herein. Sensor flex connection 123 electrically couples ISF sensing module 120 to on-patch electronics 104 and/or reusable electronics module 110.

In the exemplary embodiment, rigid support 112 includes a base 124, vertical supports 126, and a horizontal support 128. Vertical supports 126 extend from base 124, and horizontal support 128 is spaced from base 124 and extends between vertical supports 126. Vertical supports 126 each define a slot 130, and base 124 defines a plurality of grooves 132. In the exemplary embodiment, rigid support 112 is formed of a rigid material, such as, but not limited to, a rigid metal, a rigid plastic, and/or any other material that enables microneedle actuator 108 to operate as described herein. As described herein, rigid support 112 is configured to position spring 114, ram 116, geared cam 118, and ISF sensing module 120 relative to each other such that spring 114 drives into ram 116, and ram 116 rotates geared cam 118 such that ISF sensing module 120 is inserted into the user's skin.

In the exemplary embodiment, spring 114 is formed of an elastic material, such as, but not limited to, spring steel, thermoplastic polyurethane, an elastic metal, an elastic composite, an elastic plastic, and/or any other material that enables microneedle actuator 108 to operate as described herein. More specifically, spring 114 is formed of an elastic material that stores and releases an appropriate amount of energy to rotate geared cam 118 and penetrate the skin of the user with ISF sensing module 120. Spring 114 is held in tension in the stored configuration by actuation device 122 and rigid support 112. As described herein, actuation device 122 is selectively actuated such that spring 114 drives into ram 116, and ram 116 rotates geared cam 118 such that ISF sensing module 120 is inserted into the user's skin. Spring 114 may be a compression spring, an extension spring, a torsion spring, a coil spring and/or a leaf spring. In alternative embodiments, spring 114 may be any mechanical device configured to store and release energy as described herein.

Ram 116 is positioned between vertical supports 126 and between base 124 and horizontal support 128. Ram 116 has a face 134 configured to receive spring 114 and a curved side 136 configured to receive a portion of geared cam 118. Spring 114 is positioned between rigid support 112 and ram 116 such that a first end 138 of spring 114 is positioned with face 134. As described herein, after actuation device 122 has been actuated, spring 114 exerts a force on face 134 and translates ram 116 toward geared cam 118 along a direction 140 to rotate geared cam 118. Specifically, curved side 136 presses against geared cam 118, causing geared cam 118 to rotate.

Geared cam 118 translates the linear motion of spring 114 and ram 116 into rotational motion of ISF sensing module 120. In the exemplary embodiment, geared cam 118 has a body 142 having an irregular cylindrical shape, a first flat surface 144, a second flat surface 146, an axle 147 including pins 148 attached to body 142, and gear teeth 150 extending from body 142 and second flat surface 146. ISF sensing module 120 is attached to first flat surface 144. Pins 148 are cylindrical shafts that extend from body 142, and geared cam 118 is positioned between vertical supports 126 such that pins 148 extend from body 142 into slots 130. Pins 148 allow geared cam 118 to rotate and translate in direction 140 while maintaining geared cam 118 between vertical supports 126.

Gears 150 extend from body 142 and second flat surface 146 and, in the exemplary embodiment, have a generally rounded shape that complements the generally rounded shape of grooves 132. In a first configuration illustrated in FIGS. 5 and 6, at least one gear tooth 150 is positioned with at least one groove 132. In the embodiment illustrated in FIGS. 5 and 6, two gear teeth 150 are positioned within two grooves 132. Rotation of geared cam 118 is partly enabled by gear teeth 150 rotating into grooves 132 such that each gear tooth 150 and groove 132 provides a pivot point about which geared cam 118 pivots during rotation of geared cam 118. For example, as shown in FIGS. 5 and 6, geared cam 118 includes five gear teeth 150 (a first gear tooth 152, a second gear tooth 154, a third gear tooth 156, a fourth gear tooth 158, and a fifth gear tooth 160) and base 124 defines five grooves 132 (a first groove 162, a second groove 164, a third groove 166, a fourth groove 168, and a fifth groove 170). In the first configuration illustrated in FIG. 5, first gear tooth 152 is positioned within first groove 162, and geared cam 118 pivots about first gear tooth 152 as it begins rotating. Additionally, as geared cam 118 rotates, second gear tooth 154 rotates into second groove 164 and first gear tooth 152 rotates out of first groove 162 such that geared cam 118 pivots about second gear tooth 154 rather than first gear tooth 152. As geared cam 118 rotates, the pivot point moves sequentially from first gear tooth 152 to fifth tooth gear 160. Gear teeth 150 and grooves 132 provide sequential pivot points about which geared cam 118 pivots during rotation of geared cam 118. Without gear teeth 150 and grooves 132, geared cam 118 may slide without rotating such that ISF sensing module 120 is not inserted into the user's skin. Accordingly, gear teeth 150 and grooves 132 enable rotation of geared cam 118, ensuring that ISF sensing module 120 is inserted into the user's skin.

Actuation device 122 retains spring 114, ram 116, geared cam 118, and ISF sensing module 120 in the first configuration until a user activates actuation device 122, releasing spring 114, ram 116, geared cam 118, and ISF sensing module 120 as described above, and inserting ISF sensing module 120 into the user's skin. As shown in FIGS. 7 and 8, in the exemplary embodiment, actuation device 122 includes a heater 172, a first strap 174, a second strap 176, and a sensor 177. First strap 174 includes a first backing 178 and a first substrate 180 attached to first backing 178, and second strap 176 includes a second backing 182 and a second substrate 184 attached to second backing 182. First backing 178 and second backing 182 each include a first side 186, a second side 188, a first end 190, and a second end 192. First sides 186 of first backing 178 and second backing 182 face toward spring 114, ram 116, geared cam 118, and ISF sensing module 120, and second sides 188 of first backing 178 and second backing 182 face away from spring 114, ram 116, geared cam 118, and ISF sensing module 120. First end 190 of first backing 178 is attached to horizontal support 128, and second end 192 of first backing 178 is releasably attached to first end 190 of second backing 182 as described below. Second end 192 of second backing 182 is attached to geared cam 118. In an alternative embodiment, actuation device 122 does not include first backing 178 and second backing 182. Rather, first substrate 180 and second substrate 184 are directly attached to horizontal support 128 and geared cam 118 respectively. In the exemplary embodiment, first strap 174 and second strap 176 each include a porous, hydrophobic sheet, a film material, a paper, a textile, an adhesive-backed textile, a non-woven polymer sheet, a cast polymer sheet, a laser-drilled polymer sheet, and/or a supported version of any of the above.

First substrate 180 and second substrate 184 are fibrous, porous substrates attached to first side 186 of first backing 178 and second side 188 of second backing 182. In the exemplary embodiment, first substrate 180 and second substrate 184 include porous, hydrophobic sheet or film material, a paper, a textile, an adhesive-backed textile, a non-woven polymer sheet, a cast polymer sheet, a laser-drilled polymer sheet, any other porous material, and/or a supported version of any of the above and/or any other porous material. In the exemplary embodiment, first substrate 180 and second substrate 184 include a non-woven polyethylene sheet. As shown in FIG. 7, first substrate 180 and second substrate 184 are attached to first backing 178 and second backing 182 such that first substrate 180 and second substrate 184 contact each other in the first configuration. Additionally, in the exemplary embodiment, first substrate 180 and second substrate 184 are infused with an adhesive material such that first substrate 180 and second substrate 184 adhere to each other in the first configuration. Adhesion of first substrate 180 to second substrate 184 maintains spring 114, ram 116, geared cam 118, and ISF sensing module 120 in the first configuration until heater 172 melts the adhesive material, releasing spring 114, ram 116, geared cam 118, and ISF sensing module 120 as described above and inserting ISF sensing module 120 into the user's skin.

In the exemplary embodiment, the adhesive material includes any substance that adheres to first strap 174 to second strap 176 at a first predetermined temperature, preventing rotation of geared cam 118 and ISF sensing module 120, and disengages first strap 174 from second strap 176 at a second predetermined temperature, enabling rotation of geared cam 118 and ISF sensing module 120 and insertion of ISF sensing module 120 into the user's skin. For example, in the exemplary embodiment the adhesive material includes a wax. Specifically, in an embodiment the wax includes a polyolefin wax including eicosane, docosane, tetracosane, hexacosane, octacosane, triacontane, dotriacontane, tetratriacontane, hexatriacontane, octatriacontane tetracontane, dotriacontane, tetratetracontane, and/or mixtures thereof. In another embodiment the wax includes a fluoropolymer wax, a fatty acid amide wax, a Fischer-Tropsch wax, a paraffin wax, a polyolefin wax, and/or mixtures thereof. In yet another embodiment, the wax includes a biologically-derived wax such as a plant-based wax (carnuba, palm, etc.) or an animal-derived wax (tallow, beeswax, etc.).

Heater 172 is positioned on horizontal support 128 proximate the adhesive material infused into first substrate 180 and second substrate 184. Additionally, heater 172 is electrically and communicatively coupled to on-patch electronics 104 and/or reusable electronics module 110. On-patch electronics 104 and/or reusable electronics module 110 enable communication between heater 172 and the user, enabling the user to activate heater 172. In the exemplary embodiment, the adhesive material is a wax having a melting temperature of about 40 °C to about 70 °C and that melts when heater 172 increases the temperature of the wax above the melting temperature. The wax adheres to first strap 174 to second strap 176 at a first predetermined temperature, preventing rotation of geared cam 118 and ISF sensing module 120, and disengages first strap 174 from second strap 176 at a second predetermined temperature, enabling rotation of geared cam 118 and ISF sensing module 120 and insertion of ISF sensing module 120 into the user's skin. That is, the wax has a predetermined adhesion temperature (approximately the melting temperature) below which the wax adheres first strap 174 to second strap 176. However, the wax disengages first strap 174 from second strap 176 when a temperature of the wax rises from the first predetermined temperature to the second predetermined temperature such that the temperature of wax meets or exceeds the predetermined adhesion temperature.

In the exemplary embodiment, heater 172 includes a thin film heater positioned on horizontal support 128 proximate the wax infused into first substrate 180 and second substrate 184. In alternative embodiments, heater 172 may include a thick film heater, an inductively coupled heater, a resistive heater, a photothermal heater, a thermoelectric heater, a piezo heater, an RF/magnetic heater, a laser, a light source, and/or a chemical heater. In an alternative embodiment, heater 172 is formed integrally with horizontal support 128. Heater 172 is configured to selectively increase the temperature of the wax from the first predetermined temperature to the second predetermined temperature such that the temperature of the wax meets or exceeds the predetermined adhesion temperature. Sensor 177 is attached to rigid support 112 to determine a status of actuation device 122. Specifically, sensor 177 is configured to deactivate heater 172 after heater 172 increases a temperature of the adhesive material above the melting point of the adhesive material. In the exemplary embodiment, sensor 177 includes a circuit including a mechanically breakable conductive link (not shown). The mechanically breakable conductive link is broken when actuation device 122 is actuated.

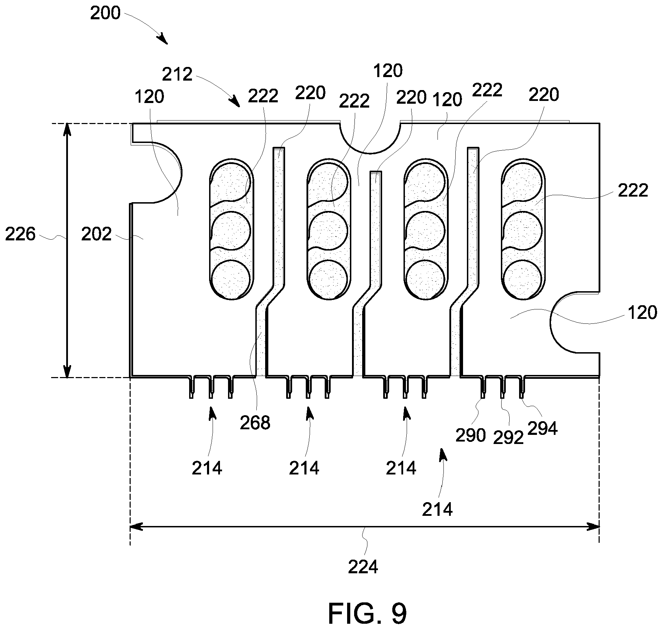

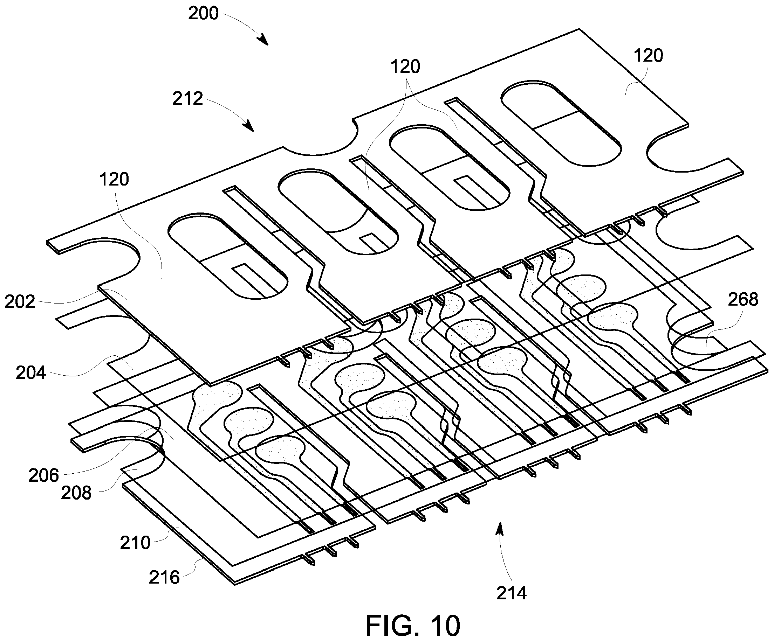

FIG. 9 is a top view of an exemplary embodiment of an ISF sensing module 200 and FIG. 10 is an exploded perspective view of ISF sensing module 200. As shown in FIGS. 9 and 10, ISF sensing module 200 is formed of a plurality of layers 202-210 laminated together to form a sensor component 212 and a plurality of microneedles 214 integrally formed with sensor component 212 for insertion into the skin of the user and sensing the ISF of the user, as described in detail below. ISF sensing module 200 is then separated into separate ISF sensing modules 120 and attached to microneedle actuators 108 as described above for sensing the ISF of the user. Sensor component 212 supports microneedles 214 during insertion into the skin of the user and adheres ISF sensing module 200 to the skin of the user, as described below. Microneedles 214 detect and measure at least one biomarker within the ISF of the user, enabling ISF sensing module 200 to monitor a medical condition and/or performance metric based on the data collected by microneedles 214. In the exemplary embodiment, ISF sensing module 200 is disposable (i.e., ISF sensing module 200 is generally used once). In an alternative embodiment, at least a portion of ISF sensing module 200 may be reusable (i.e., ISF sensing module 200 may be used for multiple uses).

ISF sensing module 200 includes a top support layer or first support layer 202, a first adhesive layer 204, a sensor layer 206, a second adhesive layer 208, and a bottom support layer or second support layer 210 laminated together to form ISF sensing module 200. Adhesive layers 204 and 208 attach top support layer 202, sensor layer 206, and bottom support layer 210. Top support layer 202 and bottom support layer 210 structurally support sensor layer 206, and sensor layer 206 senses the ISF of the user and reports the collected data to the user and/or a medical professional as described herein. Additionally, microneedles 214 are sized and shaped to enable the ISF of the user to flow to sensor layer 206 to enable sensor layer 206 to detect the ISF.

As shown in FIG. 10, top support layer 202, first adhesive layer 204, sensor layer 206, second adhesive layer 208, and bottom support layer 210 are arranged in a layered configuration and laminated. Bottom support layer 210 is the base layer that is attached to first flat surface 144 of geared cam 118. Second adhesive layer 208 is applied on top of bottom support layer 210 and adheres bottom support layer 210 to sensor layer 206 such that second adhesive layer 208 is positioned between bottom support layer 210 and sensor layer 206. First adhesive layer 204 is applied on top of sensor layer 206 and adheres sensor layer 206 to top support layer 202 such that first adhesive layer 204 is positioned between top support layer 202 and sensor layer 206. In the exemplary embodiment, first adhesive layer 204 and second adhesive layer 208 are curable adhesives that are positioned between top support layer 202, sensor layer 206, and bottom support layer 210 as described above. Specifically, in the exemplary embodiment, first adhesive layer 204 and second adhesive layer 208 are sheets of curable adhesives that are positioned between top support layer 202, sensor layer 206, and bottom support layer 210 as described above. ISF sensing module 200 is laminated by heating first adhesive layer 204 and second adhesive layer 208 until first adhesive layer 204 and second adhesive layer 208 are thermocured, bonding top support layer 202, sensor layer 206, and bottom support layer 210.

In an alternative embodiment, first adhesive layer 204 and second adhesive layer 208 are pressure sensitive adhesives that are applied to top support layer 202 and bottom support layer 210. ISF sensing module 200 is laminated by pressing top support layer 202 and first adhesive layer 204 to sensor layer 206 and bottom support layer 210 and second adhesive layer 208 to sensor layer 206 such that the pressure sensitive adhesives bond top support layer 202, sensor layer 206, and bottom support layer 210 together.

In another alternative embodiment, ISF sensing module 200 does not include first adhesive layer 204 and second adhesive layer 208. Rather, top support layer 202, sensor layer 206, and bottom support layer 210 are mechanically laminated together. For example, top support layer 202, sensor layer 206, and bottom support layer 210 are arranged in a layer configuration similar to the layer configuration illustrated in FIG. 10, and mechanical fasteners (not shown) couple top support layer 202, sensor layer 206, and bottom support layer 210 together.

Sensor component 212 of bottom support layer 210 is attached to first flat surface 144 of geared cam 118 using an adhesive surface 216 or foam. Alternatively, in some embodiments, sensor component 212 may be integrated into first flat surface 144 of geared cam 118. In the exemplary embodiment, sensor components 212 of top support layer 202 and bottom support layer 210 are formed of a pliable material, such as, but not limited to, a pliable metal, a pliable plastic, and/or any other material that enables ISF sensing module 200 to operate as described herein. In some embodiments, sensor components 212 of top support layer 202 and bottom support layer 210 are formed by additively manufacturing, casting, and/or molding a pliable metal or plastic into sensor components 212 of top support layer 202 and bottom support layer 210. In other embodiments, sensor components 212 of top support layer 202 and bottom support layer 210 are formed by machining (e.g., laser cutting) pliable metal or plastic into sensor components 212 of top support layer 202 and bottom support layer 210.

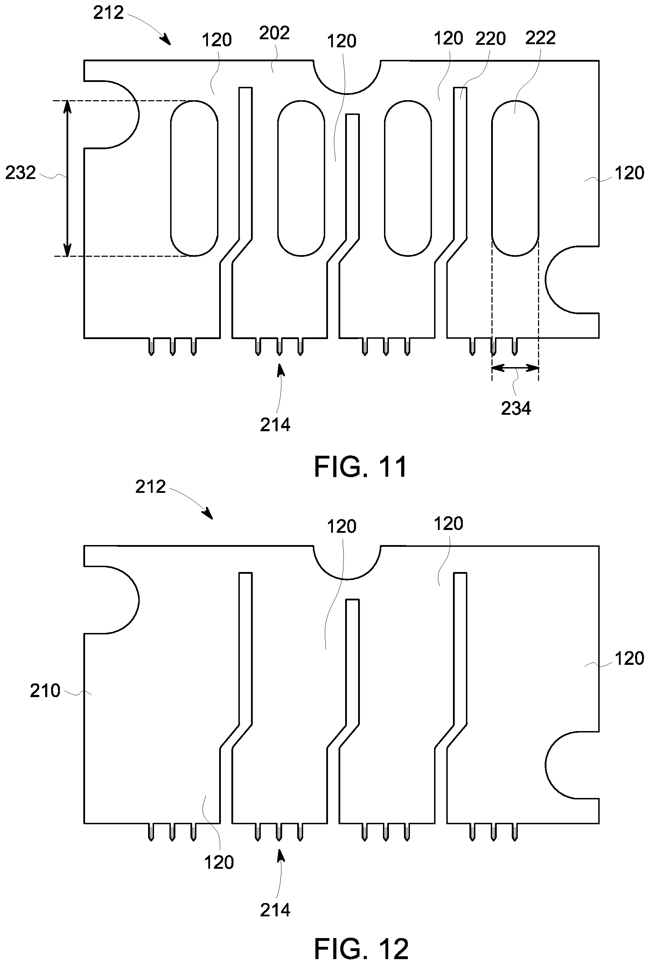

ISF sensing module 200 includes a plurality of ISF sensing modules 120 that are separable from each other such that each ISF sensing module 120 operates independently of the other ISF sensing modules 120. In the illustrated embodiment, ISF sensing module 200 includes four ISF sensing modules 120 that are separable from each other. In alternative embodiments, ISF sensing module 200 may include any number of ISF sensing modules 120 that enable ISF sensing module 200 to operate as described herein. ISF sensing modules 120 are separated from each other after layers 202-210 have been laminated together to form ISF sensing module 200. ISF sensing modules 120 are separated from each other and attached to first flat surface 144 of geared cam 118. In alternative embodiments, ISF sensing modules 120 are separated from each other by the user and/or medial professional after ISF sensing module 200 is shipped to the user, and the user and/or medial professional attaches ISF sensing modules 120 to first flat surface 144 of geared cam 118. As shown in FIGS. 9-11 and 15, top support layer 202 and bottom support layer 210 define a plurality of separation slots 220 between ISF sensing modules 120 and enable ISF sensing modules 120 to be separated from each other.

Sensor component 212 of top support layer 202 includes a plurality of cut outs 222 that enable the user and/or medical professional to access sensor layer 206 positioned between top support layer 202 and bottom support layer 210. Specifically, as shown in FIGS. 9-11, cut outs 222 are formed such that portions of sensor layer 206 are accessible by the user and/or medical professional. Cut outs 222 are sized and shaped to allow the user, the medical professional, a battery, and/or an electronic device to access sensor layer 206. In the exemplary embodiment, cut outs 222 have an oval shape. However, cut outs 222 may have any shape that enables ISF sensing module 200 to operate as described herein.

In the exemplary embodiment, sensor component 212 may have a sensor component length 224 of approximately 25 millimeters (mm) and a sensor component height 226 of approximately 13 mm, microneedles 214 may have a microneedle length 228 of approximately 800 micrometers (µm) and a microneedle width 230 of approximately 100 µm, and cut outs 222 may have a cut out length 232 of approximately 7.5 mm and a cut out width 234 of approximately 2.25 mm. Alternatively, sensor component 212, microneedles 214, and/or cut outs 222 may have any dimensions that enable ISF sensing module 200 to function as described herein. Additionally, top support layer 202 and bottom support layer 210 each have a patch layer thickness 236 of approximately 75 µm, sensor layer 206 has an sensor layer thickness 238 of approximately 25 µm, and first and second adhesive layers 204 and 208 each have an adhesive layer thickness 240 of approximately 25 µm such that sensor component 212 and microneedles 214 have a thickness 242 of approximately 325 µm.

In the exemplary embodiment, sensor component 212 has a rectangular shape. In alternative embodiments, sensor component 212 may have any shape that enables ISF sensing module 200 to operate as described herein including, without limitation, an elliptical shape, an elongated shape, a circular shape, and/or any other shape that enables ISF sensing module 200 to operate as described herein.

In the illustrated embodiment, ISF sensing modules 120 each include three microneedles 214 attached to sensor component 212. In alternative embodiment, ISF sensing modules 120 may include any number of microneedles 214 that enable ISF sensing module 200 to operate as described herein. Specifically, as described in detail below, at least one sensor 244 (i.e., an electrode) is attached to each microneedle 214 to detect biomarkers within the ISF. Some medical conditions may require that a plurality of biomarker are monitored in order to properly monitor and manage the medical condition. Accordingly, each ISF sensing module 120 may include any number of microneedles 214 and sensors 244 that enable ISF sensing module 200 to properly monitor the medical condition.

Microneedles 214 each include a microneedle base 246, a body 248, and a tip 250. Microneedle base 246 is attached to sensor component 212, body 248 is attached to microneedle base 246, and tip 250 is attached to body 248. ISF sensing module 200 is formed such that microneedle base 246 extends from sensor component 212 and is substantially coplanar with sensor component 212. Additionally, body 248 extends from microneedle base 246 and tip 250 extends from body 248 such that microneedle base 246, body 248, and tip 250 are substantially coplanar with sensor component 212.

In the exemplary embodiment, microneedles 214 each include a first surface 252, a second surface 254 opposite first surface 252, a third surface 256, and a fourth surface 258 opposite third surface 256. First surface 252 and second surface 254 each have a length that is equal to microneedle length 228 and a width that is equal to microneedle width 230. Third surface 256 and fourth surface 258 each have a length that is equal to microneedle length 228 and a width that is equal to thickness 242. In the exemplary embodiment, first surface 252, second surface 254, third surface 256, and fourth surface 258 each have a flat or planar profile. Thus, microneedles 214 each include at least one surface that has a flat or planar profile. In the exemplary embodiment, microneedles 214 each include four surfaces that each have a flat or planar profile.

In the exemplary embodiment, microneedle base 246 is formed of a pliable material to enable microneedle base 246 to be bent. In some embodiments, sensor component 212 and microneedles 214 are formed of the same pliable material. In alternative embodiments, sensor component 212 is formed of a first material and microneedles 214 are formed of a second material different than the first material. In yet another alternative embodiment, sensor component 212 and microneedles 214 may be formed of a plurality of materials. For example, sensor component 212 may be formed of the first material, microneedle base 246 may be formed of the second, pliable material, body 248 may be formed of a third material that is different than the first and second materials, and tip 250 may be formed of a fourth material that is different than the first, second, and third materials.

In the exemplary embodiment, the first, second, third, and fourth materials are the same material. Specifically, in the exemplary embodiment, the first, second, third, and fourth materials may be stainless steel or an alloy of stainless steel. In an alternative embodiment, the first, second, third, and fourth materials may be a polymer. In yet another alternative embodiment, each of the first, second, third, and fourth materials may be selected based on the function of the component they form. For example, the third and fourth materials, forming body 248 and tip 250 respectively, may be a material with a hardness (the measure of the resistance to localized plastic deformation induced by either mechanical indentation or abrasion) greater than the hardness of the second material because body 248 and tip 250 puncture the skin of the user. The second material may be a material that has greater pliability than the first, third, and fourth materials because microneedle base 246 bends.

In the exemplary embodiment, each top support layer 202 of each microneedle 214 defines a slot 260 extending through microneedle base 246 and body 248. In the illustrated embodiment, slot 260 does not extend into tip 250. In an alternative embodiment, slot 260 only extends through a portion of body 248. In another alternative embodiment, slot 260 extends through microneedle base 246, body 248, and tip 250. However, slot 260 may extend through any portion of each top support layer 202 of each microneedle 214 that enables ISF sensing module 200 to operate as described herein. Slot 260 has a slot length 262 and a slot width 264. In the exemplary embodiment, slot length 262 is substantially greater than slot width 264, and slot 260 forms a channel that channels ISF to sensors 244 for detection of biomarkers, as described herein. Slot 260 extends through top support layer 202 of microneedle 214 and, as such, slot 260 has a slot depth 266 approximately equal to patch layer thickness 236 of approximately 75 µm. In alternative embodiments, slot 260 may have any size and shape that enables ISF sensing module 200 to operate as described herein.

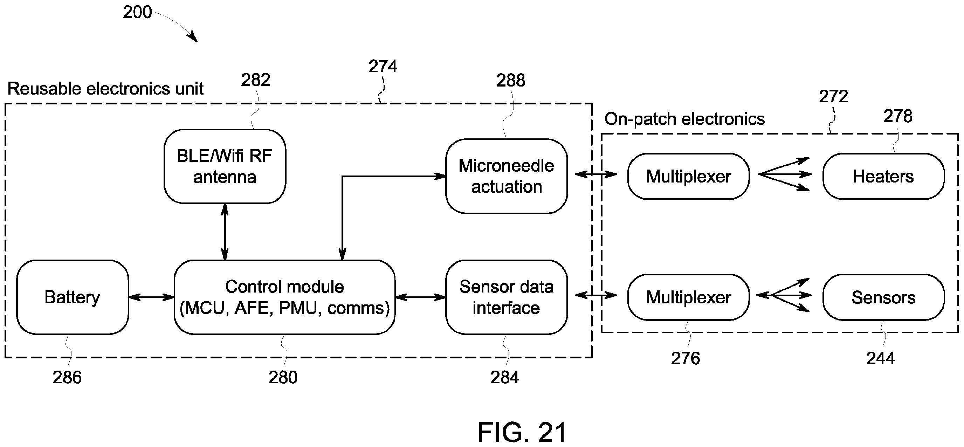

Turning now to sensor layer 206 sensor layer 206 includes a substrate 268, at least one electrical circuit 270, sensors 244, and communication and energy management components. FIG. 21 is a block diagram of an exemplary electronics architecture of the communication and energy management components. The communication and energy management components described above may include an on-patch control module 272, an off-patch control module 274, and a cable (not shown) communicatively coupling electrical circuit 270, sensors 244, and/or on-patch control module 272 to off-patch control module 274. Sensor layer 206 may include any of on-patch control module 272, off-patch control module 274, and the cable. Off-patch control module 274 may be reusable electronics module 110 and may include data storage, power management, and communications components, as described herein. On-patch control module 272 may also be referred to herein as an analog-to-digital signal module. Performing the analog to digital conversion close to sensor 244 minimizes noise and spurious signals that may interfere with signals generated by sensors 244, as well as piezoelectric voltages generated in the connection cables. The distributed configuration of sensor layer 206 enables reducing the footprint and weight of the portion of sensor layer 206 in direct contact with the skin (i.e., on-patch control module 272). Specifically, on-patch control module 272 may be relatively light-weight and have a thin form factors such that ISF sensing modules 120 is attached to geared cam 118. Further, because on-patch control module 272 is separate from off-patch control module 274, off-patch control module 274 may be positioned remotely from sensor component 212. Alternatively, on-patch control module 272 and off-patch control module 274 are incorporated in the same component, and are not located remotely from one another. As another alternative, in some embodiments, on-patch control module 272 and off-patch control module 274 are located remotely from one another, but are not communicatively coupled using the cable. Instead, in such embodiments, on-patch control module 272 and off-patch control module 274 communicate with each other wirelessly, using any suitable wireless communications protocol.

If off-patch control module 274 is not reusable electronics module 110, off-patch control module 274 may be positioned remotely from on-patch control module 272 using the cable. For example, off-patch control module 274 may be stored in a pocket of a garment (e.g., pants or shirt), or may be attached, for example, to a belt or a heart-rate monitor strap (e.g., using a clip or hook and loop fasteners). In embodiments where off-patch control module 274 is incorporated into a garment (e.g., an elastic and form-fitting garment), the cable may be a flexible conductor sewn or otherwise incorporated into the garment to allow ISF sensing module 200 to be affixed to a particular location, while off-patch control module 274 is located in an unobtrusive location (e.g., on the back of a collar or on a sleeve of a shirt, over the sternum, on the hem of pants, in a waistband, in a pocket, etc.) or distributed over other portions of the garment.

Further, in the exemplary embodiment, off-patch control module 274 includes a memory device and a microcontroller (MCU) 280. Off-patch control module 274 may also include a wireless communications unit 282 (e.g., a Bluetooth unit), a sensor data interface unit 284, a battery unit 286, and a microneedle actuation unit 288. Battery unit 286 may be rechargeable, for example, using induction charging. In some embodiments, memory device 280 may be a microSD card or other data storage device that is removably insertable into or permanently installed in off-patch control module 274.

Off-patch control module 274 may be capable of wireless communication with a remote computing device (e.g., a mobile computing device) using Bluetooth communications or alternative approaches such as direct Wi-Fi. Further, off-patch control module 274 may be capable of bi-directional communication, allowing off-patch control module 274 or the remote computing device to store and recall prior data in the event of a communications breakdown.

In some embodiments, off-patch control module 274 communicates with an associated software application installed on the computing device. The software application may include multiple functions that assist a user in using ISF sensing module 200. The software application may upload all data to a cloud storage system, include algorithms for analyzing trends in the data, and enable users to customize the data analysis. The software application may also enable data (in a raw form or subsequent to analysis) to be displayed on the computing device. For example, in some embodiments, the software application (or off-patch control module 274 itself) may analyze the biochemical data collected by ISF sensing module 200. Further, the software application may generate an alarm when the biomarkers deviate from an expected range. Further, data may be stored on off-patch control module 274 as a backup and downloaded to the computing device upon reestablishment of communications.

In the exemplary embodiment, substrate 268 includes a patch attached to sensor component 212 of top support layer 202 and bottom support layer 210. Specifically, in the exemplary embodiment, substrate 268 is formed of polyethylene terephthalate (PET) and is attached to sensor component 212 of top support layer 202 and bottom patch layer 210 as described above. Electrical circuit 270, sensors 244, and/or on-patch control module 272 may be printed on substrate 268, and substrate 268 may be attached to sensor component 212 of top support layer 202 and bottom patch layer 210. Additionally, after substrate 268 has been attached to sensor component 212 of top support layer 202 and bottom patch layer 210, at least a portion of substrate may be laminated to sensor component 212 of top support layer 202 and bottom patch layer 210 to maintain a position of electrical circuit 270, sensors 244, and/or on-patch control module 272 on substrate 268 and/or sensor component 212 of top support layer 202 and bottom patch layer 210 during use. In an alternative embodiment, ISF sensing module 200 does not include substrate 268. Rather, electrical circuit 270, sensors 244, and/or on-patch control module 272 may be printed directly on sensor component 212 of top support layer 202 and/or bottom patch layer 210.

In another alternative embodiment, electrical circuit 270, sensors 244, and/or on-patch control module 272 are printed on both sides of substrate 268 and bottom patch layer 210 is sized and shaped like top support layer 202 such that electrical circuit 270, sensors 244, and/or on-patch control module 272 are accessible on both sides of substrate 268. That is, substrate 268 may have electrical circuit 270, sensors 244, and/or on-patch control module 272 on both sides of substrate 268 such that ISF sensing module 200 is double sided, increasing the monitoring capabilities of ISF sensing module 200. Sensors 244 positioned on opposite sides of substrate 268 may be configured to monitor the same biomarker or different biomarkers.

Electrical circuit 270 communicatively couples sensors 244 to on-patch control module 272 and/or off-patch control module 274. Specifically, electrical circuit 270 may be any circuit that transmits data from sensors 244 to on-patch control module 272 and/or off-patch control module 274.

In the illustrated embodiment, each ISF sensing module 200 includes twelve microneedles 214, and each microneedle 214 includes at least one sensor 244 positioned at least partially on body 248 and tip 250. Each sensor 244 is positioned on body 248 and/or tip 250 such that sensors 244 are embedded in the dermis of the user's skin when microneedles 214 are inserted into the user's skin. In the exemplary embodiment, each sensor 244 includes a bio sensor for sensing a biomarker within the ISF. Specifically, sensors 244 detect and measure metabolites, small molecules, proteins, ions, electrolytes, enzymes, hormones, one or more biochemical parameters, and/or any other biomarker that assists with monitoring and control of a medical condition and/or the performance of an athlete.

In the illustrated embodiment, each ISF sensing modules 120 includes three microneedles 214 and each microneedle 214 includes at least one sensor 244 such that sensor layer 206 of each ISF sensing modules 120 includes a first sensor 290, a second sensor 292, and a third sensor 294. In some embodiments, first, second, and third sensors 290, 292, and 294 are the same sensors for detecting and measuring the same biomarkers. More specifically, first, second, and third sensors 290, 292, and 294 may be configured to measure the same biochemical species and report the results (either the data from all three sensors 290, 292, and 294 or an average of all three sensors 290, 292, and 294) to the user and/or medical professional. In alternative embodiments, first, second, and third sensors 290, 292, and 294 are different sensors for detecting and measuring the different biomarkers.

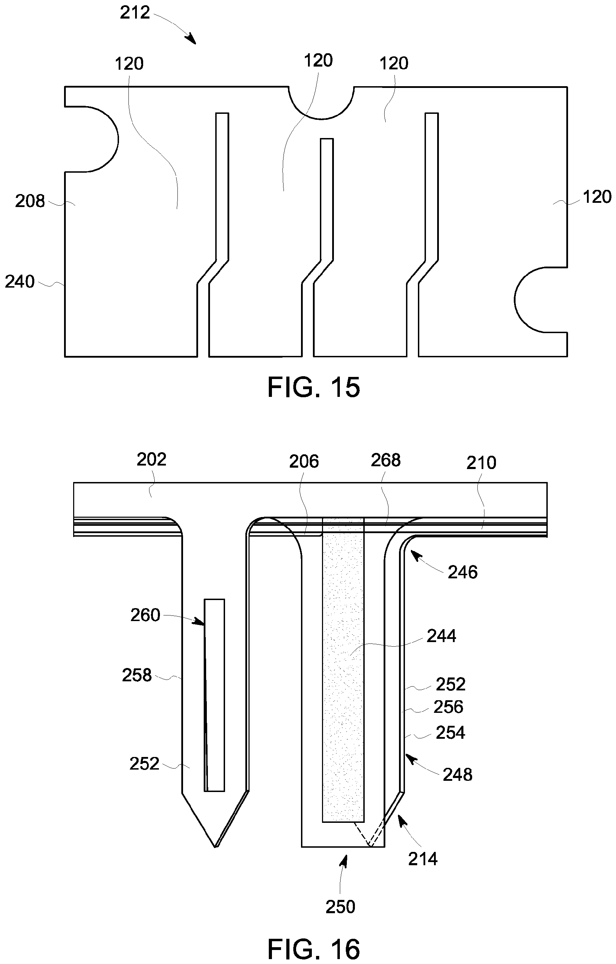

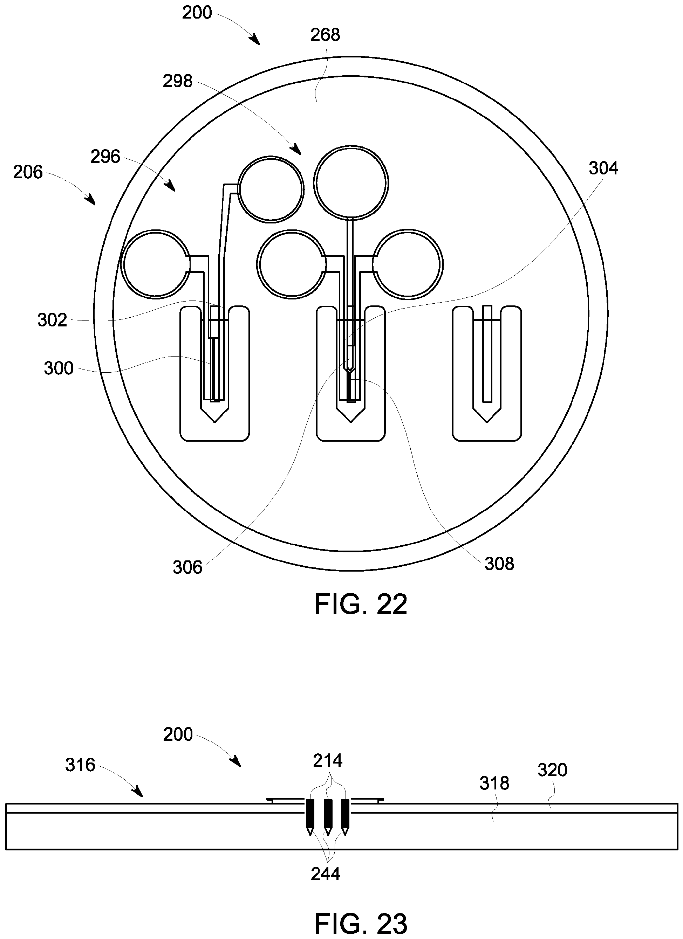

FIG. 22 is an electrical diagram of sensors 244 printed on substrate 268. In the embodiment illustrated in FIG. 15, each sensor 244 includes a 2-electrode and/or a 3-electrode biosensor that detect biomarkers within the ISF. More specifically, sensor layer 206 may include a 2-electrode biosensor 296 and/or a 3-electrode biosensor 298. 2-electrode biosensor 296 is a 2-electode system including a working electrode 300 and a counter/reference electrode 302 for detecting a specific biomarker. 3-electrode biosensor 298 is a 3-electode system including a working electrode 304, a reference electrode 306, and a counter electrode 308 for detecting a specific biomarker. 2-electrode biosensor 296 and/or 3-electrode biosensor 298 may detect ISF using a plurality of electroanalytical detection methods including, but not limited to, chronoamperometry, cyclic voltammetry, square wave voltammetry, and/or any other electroanalytical detection method that enables sensors 244 to operate as described herein. Additionally, the biomarker that is to be detected at least partially determines the type of sensor 244 embedded on microneedles 214. Additionally, sensors 244 may include other types and/or classes of biosensors. For example, sensors 244 may include field-effect transistor biosensor (not shown) and/or any other type of sensor that enables ISF sensing module 200 to operate as described herein.

For each of the biosensors described herein, working electrodes 300 and 304 include a biorecognition element (BRE) (not shown). For example, when working electrodes 300 and 304 detect metabolites, the BREs include metabolite sensing enzymes immobilized on a surface (not shown) of working electrodes 300 and 304. The choice of BREs, and their kinetics, are key to enabling continuous equilibration with biomarkers within the ISF, where high binding affinities (necessary to sense low concentrations) often correlate with poor reversibility (multiple sensor use). Antibodies, short peptides, aptamer- and non-natural peptide-based BREs may also be used to achieve target on/off equilibration rates compatible with the kinetics of target biomarker concentration fluctuations in vivo. Aptamer and short peptide BREs, in lieu of more commonly used antibodies, may selectively and specifically recognize protein and hormone biomarkers. The use of aptamers and peptides as recognition elements will decrease the cost and increase the simplicity and efficacy of biosensor fabrication. In particular, peptide and aptamer BREs enable target analyte binding in the close vicinity of the sensor surface (compared to antibodies, which are considerably larger), thus providing an effective means to overcome challenges associated with sensing in conductive biological media, such as ISF. Furthermore, aptamer and peptide BREs are considerably more stable than commonly used antibodies, offering clear advantages for in vivo monitoring in biological fluids. Other examples of BREs include non-natural peptides which offer thermal and enzymatic stabilities.

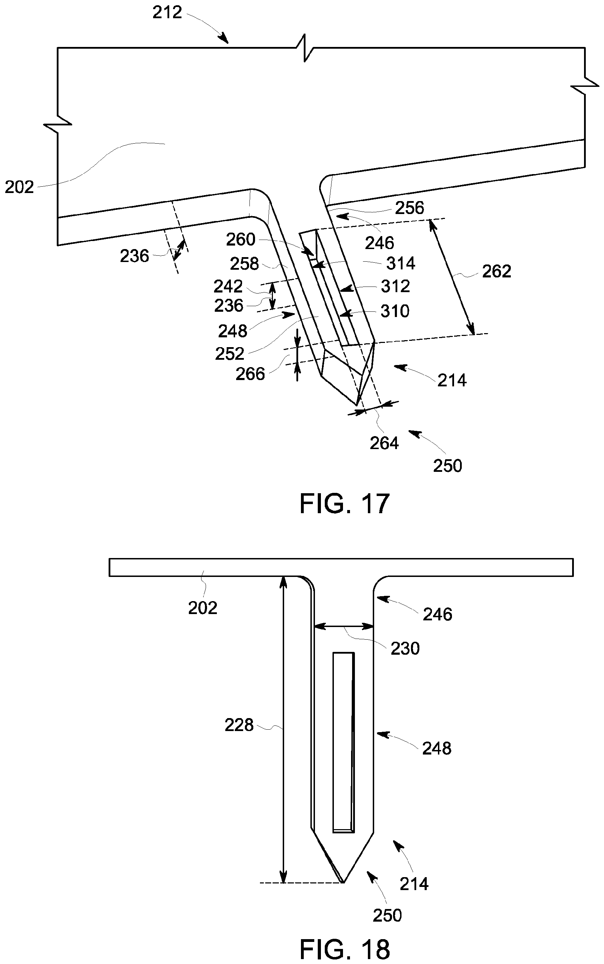

As shown in FIGS. 9, 10, and 16, substrate 268 is positioned on sensor component 212 such that electrodes 300, 302, 304, 306, and/or 308 are positioned within slot 260, and substrate 268 and slot 260 define a channel 310 for channeling ISF to electrodes 300, 302, 304, 306, and/or 308. As shown in FIG. 17, slot 260 extends through body 248 of top support layer 202 forming a first opening 312 and a second opening 314. As shown in FIG. 16, substrate 268 is positioned on sensor component 212 such that second opening 314 is covered by substrate 268. First opening 312 remains uncovered and allows ISF to flow into channel 310. More specifically, the pressure of the ISF causes the ISF to flow into channel 310 and to electrodes 300, 302, 304, 306, and/or 308 to be detected and measured. Because microneedles 214 penetrate the skin of the user, the cells adjacent microneedles 214 may be irritated by microneedles 214 and/or sensors 244. The cells proximate sensors 244 may react to the irritation by producing different biomarkers that may alter the ISF proximate microneedles 214. Accordingly, if sensors 244 were located directly adjacent to the irritated cells, the measurements reported by sensors 244 may have an error caused by the reaction of the irritated cells.

The slotted design of microneedles 214 reduces the error caused by the irritated cells. Specifically, electrodes 300, 302, 304, 306, and/or 308 are positioned within slot 260 and channel 310 and are not located directly adjacent to the irritated cells. Additionally, because the ISF flows into channel 310, the ISF detected by electrodes 300, 302, 304, 306, and/or 308 includes ISF surrounding cells that are adjacent to and remote from microneedles 214. Thus, electrodes 300, 302, 304, 306, and/or 308 detect an average biomarker concentration from cells whose position relative to microneedles 214 varies. Moreover, channeling the ISF into channel 310 enables electrodes 300, 302, 304, 306, and/or 308 to detect and measure ISF surrounding different types of cells within the dermis. Thus, the slotted design of microneedles 214 reduces the error caused by the irritated cells by channeling ISF into electrodes 300, 302, 304, 306, and/or 308.

FIG. 23 is a diagram of ISF sensing module 200 positioned on the user's skin 316 with microneedles 214 inserted into a dermis 318 of the user's skin 316. During operation, the user attaches ISF sensing module 200 to their skin 316 such that microneedles 214 puncture and penetrate an epidermis 320 and dermis 318 of user's skin 316. Specifically, sensors 244 are embedded in dermis 318 of user's skin 316 when microneedles 214 are inserted into user's skin 316 by microneedle actuator 108 as described above. Once inserted into dermis 318, ISF from the vicinity of the cells surrounding microneedles 214 flows into channel 310 and contacts electrodes 300, 302, 304, 306, and/or 308. Electrodes 300, 302, 304, 306, and/or 308 detect and measure at least one specific biomarker within the ISF. Electrodes 300, 302, 304, 306, and/or 308 then send an electrical signal to on-patch control module 272 and/or off-patch control module 274. On-patch control module 272 and/or off-patch control module 274 may analyze the data from electrodes 300, 302, 304, 306, and/or 308 and/or may transmit the data from electrodes 300, 302, 304, 306, and/or 308 to the computing device. The user and/or the medical professional may then analyze the data to determine if further medical care is required.

Because sensors 244 are embedded in dermis 318, the ISF that flows to sensors 244 is continually replenished and mechanical mechanisms to replenish the ISF are not required to enable ISF sensing module 200 to operate as described herein. Additionally, embedding sensors 244 in dermis 318 enables ISF sensing module 200 to detect biomarkers without drawing fluid out of the body of the user, reducing the cleanup and disposal of bodily fluids as the result of biomarker detection and measurement, and reducing the equipment required to measure biomarkers within the ISF. Moreover, because the pressure of the ISF causes the ISF to flow into channel 310, a fluid motive device, such as a vacuum pump, is not required to continually replenish the ISF flowing to electrodes 300, 302, 304, 306, and/or 308. Thus, ISF sensing module 200 enables detection of biomarkers while reducing cleanup and disposal of bodily fluids and reducing the equipment required to measure biomarkers within the ISF.

After a predetermined amount of time, the BREs within sensors 214 may be consumed such that sensors 214 no longer detect biomarkers within the ISF. In order to continue monitoring biomarkers within the ISF, at least one of the remote computing device, on-patch electronics 104, and/or reusable electronics module 110 activates at least one other microneedle actuator 108 of the plurality of microneedle actuator 108 and wearable ISF sensing device 100 continues to monitor the user's medical condition. Microneedle actuators 108 may be sequentially activated until all ISF sensing modules 120 of microneedle actuators 108 have been consumed. Once all ISF sensing modules 120 have been consumed, the user removes any reusable portions of wearable ISF sensing device 100 and disposes of the remainder of wearable ISF sensing device 100. The user then attaches a second wearable ISF sensing device 100 to their skin and attaches any reusable portions of wearable ISF sensing device 100 to second wearable ISF sensing device 100. The user then operates second wearable ISF sensing device 100 as described herein.

Because wearable ISF sensing device 100 includes a plurality of microneedle actuator 108 each including an ISF sensing module 120, wearable ISF sensing device 100 is capable of monitoring the user's medical condition for a longer period of time as compared to bio-sensing devices with a single ISF sensing module 120. Accordingly, a user is not required to replace wearable ISF sensing device 100 as often as other bio-sensing devices. Additionally, wearable ISF sensing device 100 described herein enable consistent monitoring of ISF because wearable ISF sensing device 100 may automatically activate another microneedle actuator 108 after sensors 214 of a first ISF sensing module 120 have been consumed. As such, wearable ISF sensing device 100 described herein enables consistent monitoring of a user's medical condition. Moreover, because wearable ISF sensing device 100 includes a plurality of microneedle actuator 108 each including a ISF sensing module 120, wearable ISF sensing device 100 reduces the number of bio-sensing devices a user consumes to monitor a medical condition.

The embodiments described herein include systems and methods for wearable ISF sensing devices. The wearable ISF sensing device includes a sensor component and a plurality of microneedle actuators positioned on the sensor component. The sensor component is attached to the skin of a user. The microneedle actuators each include a rigid support, a spring, a ram, a geared cam, an ISF sensing module, and an actuation device. The ISF sensing module is attached to the geared cam, and the ram, the spring, and the actuation device are configured to rotate the geared cam to insert the ISF sensing module into the skin of the user. Specifically, the actuation device is configured to maintain the spring in a stored configuration until selectively actuated. Once the actuation device has been actuated, the spring is released from the stored configuration to push, press, or drive the ram against the geared cam, rotating the geared cam. Rotation of the geared cam causes the ISF sensing module to rotate toward the skin of the user, inserting the ISF sensing module into the skin of the user. The ISF sensing module then monitors biochemical markers of the user.

The ISF sensing module includes a plurality of layers laminated together to form a sensor component and at least one microneedle extending from the sensor component. The layers include a first support layer, a first adhesive layer, an sensor layer, a second adhesive layer, and a second support layer. The first and second support layers structurally support the sensor layer, and the first and second adhesive layers laminate the first and second support layers to the sensor layer. Lamination of the layers ensures that the microneedles remain intact as they are inserted into the skin of the user. As such, the microneedles of the ISF sensing modulees described herein are structurally stronger than previous microneedles and remain intact as they monitor biomarkers within the user's ISF.

The microneedle includes a microneedle base, a body, and a tip. The microneedle has a substantially flat or planar profile and a slot is defined within the body of the microneedle. The slot has a first opening and a second opening. The sensor layer includes a sensor positioned over the second opening such that the sensor and the slot define a channel that channels ISF to the sensor. The microneedle and the sensor are inserted into the user's skin such that the sensor is positioned within the dermis of the user's skin. The ISF flows into the channel from adjacent tissue, and the sensor detects and measures biomarkers within the ISF. The user, observer, trainer, coach, and/or a medical professional may then monitor a medical condition or a performance/readiness metric based on the data collected by the sensor.

Because the sensors are embedded in the dermis, the ISF that flows to the sensors is continually equilibrated with the surrounding ISF without requiring mechanical mechanisms to replenish the ISF. Additionally, embedding the sensors in the dermis enables the ISF sensing module to detect biomarkers without drawing fluid out of the body of the user, reducing the cleanup and disposal of bodily fluids as the result of biomarker detection and measurement. Moreover, capillary pressure and the compressive force generated during insertion will cause ISF to flow into the channel such that a fluid motive device, such as a vacuum pump or mechanical compression, is not required to replenish the ISF at the sensors. After insertion, passive diffusion from the ISF and equilibration with the ISF inside the channel replenishes the ISF at the sensors. Thus, the ISF sensing module enables detection of biomarkers while reducing cleanup and disposal of bodily fluids and reducing the equipment required to measure biomarkers within the ISF. Additionally, directly measuring ISF in the interstitial space reduces the lag time between changes in biomarker concentration and read-out.

Because the wearable ISF sensing device includes a plurality of microneedle actuators, the wearable ISF sensing device is capable of monitoring the user's medical condition for a longer period of time compared to bio-sensing devices with a single set of microneedles. Accordingly, a user is not required to replace the wearable ISF sensing device as often as other bio-sensing devices. Additionally, the wearable ISF sensing devices described herein enable consistent monitoring of ISF because the wearable ISF sensing device may automatically activate another microneedle actuator after the sensors of a first microneedle actuator have been consumed. As such, the wearable ISF sensing devices described herein enable consistent monitoring of a user's medical condition. Moreover, because the wearable ISF sensing device includes a plurality of microneedle actuators, the wearable ISF sensing device reduces the number of bio-sensing devices a user consumes to monitor a medical condition.

An exemplary technical effect of the methods, systems, and apparatus described herein includes at least one of: (a) detecting biomarkers within ISF; (b) reducing cleanup and disposal of bodily fluids associated with biomarker detection; (c) reducing the equipment required to measure biomarkers within the ISF, and (d) reducing the error associated with detection of biomarkers within ISF.

Exemplary embodiments for wearable ISF sensing devices are described herein. The systems and methods are not limited to the specific embodiments described herein, but rather, components of systems and/or steps of the methods may be utilized independently and separately from other components and/or steps described herein. For example, the methods may also be used in combination with other systems, and are not limited to practice with only the systems described herein. Rather, the exemplary embodiment can be implemented and utilized in connection with many other systems.

Although specific features of various embodiments of the disclosure may be shown in some drawings and not in others, this is for convenience only. In accordance with the principles of the disclosure, any feature of a drawing may be referenced and/or claimed in combination with any feature of any other drawing.

This written description uses examples to disclose the embodiments, including the best mode, and also to enable any person skilled in the art to practice the embodiments, including making and using any devices or systems and performing any incorporated methods. The patentable scope of the disclosure is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A microneedle actuator (108) for monitoring interstitial fluid (ISF) of a user, said microneedle actuator (108) comprising:
a rigid support (112) defining a slot (130);
a geared cam (118) positioned within said rigid support (112) and comprising an axle (147) extending through the slot (130);
a spring (114) positioned within said rigid support (112) and configured to rotate said geared cam (118) about said axle (147);
an ISF sensing module (120) attached to said geared cam (118) and comprising a plurality of microneedles (214), each microneedle of said plurality of microneedles (214) comprising at least one sensor (244) for detecting the ISF of the user; and
an actuation device (122) attached to said rigid support (112) and said geared cam (118), wherein actuation of said actuation device (122) releases said spring (114), rotating said geared cam (118) to insert said plurality of microneedles (214) into the user's skin, and wherein said at least one sensor (244) detects constituents of the ISF of the user.

2. The microneedle actuator (108) in accordance with Claim 1, wherein said spring (114) comprises at least one of a compression spring, an extension spring, a torsion spring, a coil spring, and a leaf spring.

3. The microneedle actuator (108) in accordance with Claim 1 further comprising a ram (116) positioned within said rigid support (112) between said spring (114) and said geared cam (118), wherein actuation of said actuation device (122) releases said spring (114), causing said spring (114) to press on said ram (116), causing said ram (116) to rotate said geared cam (118), and causing said geared cam (118) to insert said plurality of microneedles (214) into the user's skin, and wherein said ram (116) comprises a curved surface (136) configured to receive a portion of said geared cam (118) and a face (134) configured to receive said spring (114).

4. The microneedle actuator (108) in accordance with Claim 1, wherein said geared cam (118) comprises a body (142), a first flat surface (144), a second flat surface (146), and a plurality of gear teeth (150) extending from said body (142) and said second flat surface (146).

5. The microneedle actuator (108) in accordance with Claim 4, wherein said ISF sensing module (120) is attached to said first flat surface (144).

6. The microneedle actuator (108) in accordance with Claim 4, wherein said rigid support (112) defines a plurality of grooves (132), and wherein rotation of said geared cam (118) rotates a gear tooth of said plurality of gear teeth (150) into a groove (132) of said plurality of grooves (132).

7. The microneedle actuator (108) in accordance with Claim 6, wherein each gear tooth (150) within each groove (132) defines a pivot point about which said geared cam (118) pivots during rotation of said geared cam (118).

8. The microneedle actuator (108) in accordance with Claim 1, wherein said rigid support (112) defines a plurality of grooves (132) embossed in said rigid support (112) along a first plane, and wherein the slot (130) is a pair of slots that are defined within said rigid support (112) to cooperatively define a second plane extending through the pair of slots (130) that is parallel to the first plane.

9. The microneedle actuator (108) in accordance with Claim 8, wherein said geared cam (118) comprises a body (142) and a plurality of gear teeth (150) extending from said body (142), and wherein a first distance between a center-line of the pair of slots (130) and the first plane is the same as a radius of the body (142) of the geared cam (118).

10. The microneedle actuator (108) in accordance with Claim 1, wherein said actuation device (122) comprises a first strap (174) and a second strap (176), said first strap (174) and said second strap (176) are infused with an adhesive material such that said first strap (174) and said second strap (176) adhere to each other, wherein said adhesive material comprises at least one of a fluoropolymer wax, a fatty acid amide wax, a Fischer-Tropsch wax, a paraffin wax, a biological wax, a polyolefin wax, and/or a polyolefin wax including eicosane, docosane, tetracosane, hexacosane, octacosane, triacontane, dotriacontane, tetratriacontane, hexatriacontane, octatriacontane tetracontane, dotriacontane, and/or tetratetracontane, and wherein said first strap (174) and said second strap (176) each comprise a porous, hydrophobic sheet, a film material, a paper, a textile, an adhesive-backed textile, a non-woven polymer sheet, a cast polymer sheet, and/or a laser-drilled polymer sheet.

11. A microneedle actuator (108) for monitoring interstitial fluid (ISF) of a user, said microneedle actuator (108) comprising:
a rigid support (112) defining a slot (130);
a geared cam (118) positioned within said rigid support (112) and comprising an axle (147) extending through the slot (130);
a spring (114) positioned within said rigid support (112) and configured to rotate said geared cam (118) about said axle (147);
an ISF sensing module (120) attached to said geared cam (118), said ISF sensing module (120) comprising:
a first support layer (202);
a sensor layer (206) laminated to said first support layer (202) to form a sensor component (212); and
a plurality of microneedles (214) extending from said sensor component (212), said sensor layer (206) comprising a substrate (268) and at least one sensor (290) positioned on said substrate (268), each microneedle of said plurality of microneedles (214) comprising:
a microneedle base (246) extending from said sensor component (212);
a body (248) extending from said microneedle base (246), said body (248) defining a slot (130) extending through said first support layer (202) of said body (248), the slot (130) defining a first opening (312) and a second opening (314), said at least one sensor (290) positioned over the second opening (314), wherein the first opening (312), the slot (130), and said at least one sensor (244) define a channel (310) exposed to the environment; and
a tip extending from said body (248), wherein the channel (310) is configured to channel (310) ISF to said at least one sensor (244); and
an actuation device (122) attached to said rigid support (112) and said geared cam (118), wherein actuation of said actuation device (122) releases said spring (114), rotating said geared cam (118) to insert said plurality of microneedles (214) into the user's skin, and wherein said at least one sensor (244) detects the ISF of the user.

12. The microneedle actuator (108) in accordance with Claim 11, wherein said ISF sensing module (120) further comprises a second support layer (210) laminated to said first support layer (202) and said sensor layer (206), wherein said sensor layer (206) is positioned between said first support layer (202) and said second support layer (210), and wherein said first support layer (202) and said second support layer (210) structurally support said sensor layer (206).

13. The microneedle actuator (108) in accordance with Claim 12, wherein said ISF sensing module (120) further comprises a second support layer (210) laminated to said sensor layer (206), a first adhesive layer (204) positioned between said first support layer (202) and said sensor layer (206), and a second adhesive layer (208) positioned between said second support layer (210) and said sensor layer (206), and wherein said first adhesive layer (204) laminates said first support layer (202) to said sensor layer (206).

14. The microneedle actuator (108) in accordance with Claim 13, wherein said first adhesive layer (204) and said second adhesive layer (208) each comprise a sheet of a curable adhesive.

15. The microneedle actuator (108) in accordance with Claim 13, wherein said first adhesive layer (204) and said second adhesive layer (208) each comprise a pressure sensitive adhesive.
